# FASCICULE DE BREVET EUROPEEN

(11) **EP 1 124 633 B1**
(45) Date de publication et mention de la délivrance du brevet: **22.01.2003**
(21) Numéro de dépôt: 99950867.4
(22) Date de dépôt: 28.10.1999
(51) Int. Cl.: B01J 20/32, B01D 15/08, A61M 1/36

(54) **UTILISATION D'UN GEL ADSORBANT POUR ELIMINER ET PURIFIER LES BIOMOLECULES**
VERWENDUNG EINES ADSORBIERENDEN GELS ZUR ENTFERNUNG UND REINIGUNG VON BIOMOLEKÜLEN
USE OF AN ADSORBENT GEL FOR ELIMINATING AND PURIFYING BIOMOLECULES

(30) Priorité: 30.10.1998 FR 9813655
(43) Date de publication de la demande: 22.08.2001
(73) Titulaire: CENTRE NATIONAL DE LA RECHERCHE SCIENTIFIQUE, 75016 Paris Cédex (FR)
(72) Inventeur: VIJAYALAKSHMI, Mookambeswaran, F-60200 Compiegne (FR); PITIOT, Olivier, F-02200 Soissons (FR); LEGALLAIS, Cécile, F-60340 Villers sous Saint Leu (FR); MORINIERE, Philippe, F-80000 Amiens (FR)
(74) Mandataire: Goulard, Sophie
(86) Numéro de dépôt international: FR9902635
(87) Numéro de publication internationale: WO00025911

(56) Documents cités:
- EP-A- 0 295 073
- EP-A- 0 510 393
- WO-A-90/12803
- US-A- 4 177 038
- US-A- 4 721 730
- US-A- 4 770 774
- J. PORATH: "adsorptive size exclusion chromatography (concentration) Adsec" INT. J. OF BIOCHROMATOGRAPHY, vol. 3, no. 1, 1997, pages 9-17, XP002110958 cité dans la demande
- PORATH J ET AL: "METAL CHELATE AFFINITY CHROMATOGRAPHY, A NEW APPROACH TO PROTEIN FRACTIONATION" NATURE, vol. 258, 18 décembre 1975 (1975-12-18), page 598/599 XP002059246 ISSN: 0028-0836 cité dans la demande

## Description

La présente invention est relative à l'utilisation d'un gel adsorbant alliant les propriétés des chromatographies d'exclusion stérique et d'affinité (gel AdSEC, pour "*Adsorptive Size Exclusion Chromatography*").

Le principe d'un gel AdSEC résulte de la fusion de deux techniques chromatographiques : exclusion de taille et affinité, afin d'obtenir des supports alliant les propriétés les plus intéressantes de ces dernières.

La chromatographie d'exclusion de taille (filtration sur gel) permet la séparation des molécules en fonction de leur seul encombrement stérique lors de leur diffusion passive dans un tamis moléculaire (gel). Les plus grosses molécules ne peuvent pénétrer la matrice réticulée et sont par conséquent exclues plus rapidement de la colonne. Cette technique possède la particularité de ne pas présenter d'interactions entre le support et les molécules, donc d'être relativement peu sensible aux conditions biochimiques (pH, force ionique) de la solution. En revanche, du fait de son principe de diffusion, les facteurs limitants de son utilisation sont généralement un temps de manipulation important (car on utilise des débits faibles), ainsi qu'un dépôt d'échantillons relativement restreint (1 à 5 % du volume de la colonne).

La chromatographie d'affinité repose sur des interactions moléculaires entre le support (matrice sur laquelle on greffe des ligands d'affinité) et les molécules à séparer. Parmi ces ligands d'affinité, les ions métalliques immobilisés, introduits en 1975 par Porath *et al.* (Nature, 1975, 21, 598-599), représentent une méthode de séparation basée sur les interactions (liaisons de coordination) entre des biomolécules en solution et des ions métalliques immobilisés sur un support ; les ions Zn(II), Cu(II), Ni(II) et Co(II) sont les plus couramment utilisés. On parle de chromatographie d'affinité sur ions métalliques immobilisés (IMAC).

L'utilisation conjointe des principes des chromatographies d'exclusion stérique et d'affinité (AdSEC) a été discutée par Porath *et al.* (Int. J. of Bio-Chromatogr., 1997, 3, 9 - 17). Ces auteurs ont montré que des dérivés iminodiacétiques de dextran portant des ions métalliques comme ligand d'affinité permettent une exclusion stérique et sont susceptibles de concentrer efficacement des solutions par leur propriétés d'adsorption et d'affinité. Ces auteurs ont montré qu'une colonne de gel AdSEC d'un volume de 5 ml pouvait fixer un pourcentage important de composés ayant un poids moléculaire compris entre 5 kDa et 50 kDa et les concentrer environ 1000 fois en une seule opération.

De tels supports permettent d'adsorber les plus petites molécules (ayant une affinité pour le ligand greffé) à des débits et des volumes importants (non autorisé en filtration sur gel). D'autre part lors de la synthèse du gel adsorbant, le seuil d'accessibilité au ligand d'affinité peut être modulé durant la synthèse du gel en fonction de la taille de la biomolécule à éliminer ou à purifier.

L'insuffisance rénale terminale touche actuellement 22000 personnes en France dont 20000 sont traitées par hémodialyse itérative. Seulement 1800 peuvent espérer être transplantées chaque année, sachant qu'un quart d'entre elles reviendra dans les cinq ans en hémodialyse en raison d'un rejet pour attendre une nouvelle transplantation.

La survie de l'urémique, toutes méthodes confondues peut dépasser 25 ans s'il n'est pas porteur d'affection cardio-vasculaire sévère. Dans ce cas, sa qualité de survie se trouve profondément altérée au fil des ans par les complications ostéoarticulaires de l'urémie terminale, au premier plan desquelles sont décrites les arthropathies érosives secondaires aux dépôts de la β2-microglobuline (β2-M).

Le mécanisme de survenue de ces arthropathies commence dès qu'apparaît l'insuffisance rénale responsable d'une accumulation de β2-microglobuline. Cette protéine de poids moléculaire de 11800 Da, va s'accumuler dans l'organisme au fil des années et se déposer sélectivement au niveau des disques cervicaux, des épaules, des hanches et des poignets. Des dépôts cardiaques et digestifs ont été rapportés. Ces dépôts vont fragiliser l'articulation et l'os adjacent jusqu'à détruire totalement l'articulation. Ainsi, on observe un effondrement des corps vertébraux pouvant entraîner une compression médullaire avec perte de commande des quatre membres, des luxations articulaires irréversibles, une perte de la préhension au niveau des mains et des pseudo fractures de la hanche. Des compressions nerveuses canalaires sont observées comme le syndrome du canal carpien.

Ces complications conduisent irrémédiablement l'urémique vers l'invalidité et l'état grabataire que ne peuvent prévenir les méthodes de dialyse classique. La transplantation permet une stabilisation de ces lésions.

Afin de prévenir efficacement ces complications, il importe de pouvoir épurer efficacement les composants polluants du sang, en particulier la β2-microglobuline, qui sont synthétisés quotidiennement par l'organisme et qui ne sont pas ou pas suffisamment éliminés par les reins défectueux chez les patients dialysés.

L'épuration de ces différentes biomolécules ne peut se faire qu'au niveau des membranes artificielles lors de la dialyse qui actuellement sont insuffisamment efficaces malgré une épuration par filtration et adsorption non spécifique membranaire.

Les techniques existantes pour l'élimination des biomolécules dont la β2-microglobuline sont actuellement de 3 types :

### 1. Elimination des biomolécules par hémodialyse

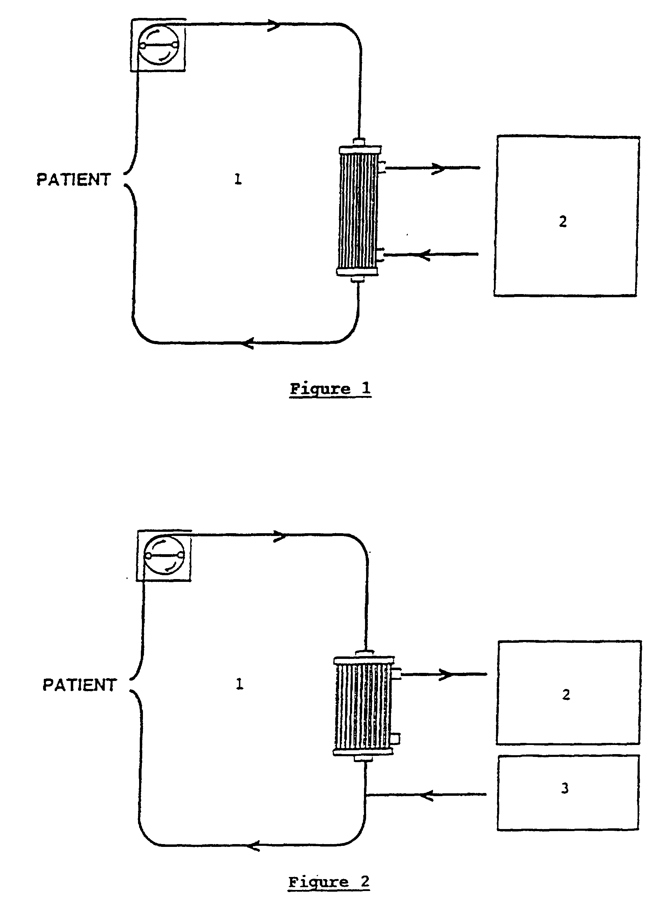

L'hémodialyse est une technique destinée à des sujets atteints d'une insuffisance rénale partielle ou totale (figure 1). Elle consiste en un traitement extracorporel du sang, assurant les mêmes fonctions que le rein grâce à un procédé membranaire. La partie essentielle de l'hémodialyseur (1) est une membrane d'échange, de part et d'autre de laquelle, circulent à contre courant le sang du patient et le dialysat issu du générateur d'hémodialyse (2). Cette technique permet l'épuration des composés de petit poids moléculaire polluant le sang, tels que l'urée, les acides aminés, les sels minéraux qui sont normalement éliminés par le rein. Dans le cas de la β2-microglobuline sérique, les différentes membranes de dialyse couramment utilisées possèdent deux propriétés antagonistes :
- capture de la β2-microglobuline par adsorption non-spécifique sur la membrane,
- génération de la β2-microglobuline par décrochage de cette molécule qui est associée de façon non covalente à la surface des cellules nucléées sanguines dans le complexe majeur d'histocompatibilité de type I.

Le degré de génération de β2-microglobuline est un des critères qui définissent la biocompatibilité des membranes. Ainsi fort de ces deux propriétés antagonistes, certaines membranes conduisent lors d'une séance d'hémodialyse globalement à une augmentation de la concentration en β2-microglobuline, alors que d'autres la diminuent.

Cependant, quelles que soient les membranes utilisées, ces résultats sont nivelés sur des périodes supérieures à un an. Ainsi, il a été constaté que le taux plasmatique de la β2-microglobuline chez les patients urémiques après quinze mois de dialyse était invariablement augmenté pour être compris entre 40 et 50 mg/l (contre 1 à 2 mg/l chez les patients sains). De tels problèmes de biocompatibilité se retrouvent également pour les autres biomolécules.

### 2. Elimination des biomolécules par hémofiltration

Une fois par mois, le dialysé subit une séance d'ultrafiltration. Le module utilisé (1) possède un seuil de coupure plus élevé qu'en hémodialyse (seuil moyen de 40 kDa) et permet l'élimination par filtration des petites molécules du plasma, dont les plus petites protéines, telle que la β2-microglobuline (figure 2). Durant une séance d'ultrafiltration, la perte d'eau plasmatique est compensée par un apport équivalent en liquide physiologique (3).

Les résultats qualitatifs, vis à vis de l'élimination de la β2-microglobuline (épuration et génération de cette molécule par les membranes d'ultrafiltration), sont similaires à ceux obtenus en hémodialyse. On retrouve ainsi une influence importante de la nature de la membrane et de la durée de l'hémofiltration. Si certaines membranes semblent éliminer plus de β2-microglobuline sur 5 heures (une séance), on assiste également à un nivellement des résultats au cours du temps. Au niveau quantitatif, il semble qu'environ 50% de la β2-microglobuline sérique soit éliminée par séance d'hémofiltration. Cependant, même si cette technique est plus efficace pour l'épuration de la β2-microglobuline que l'hémodialyse, elle reste insuffisante pour prévenir et empêcher l'apparition de la maladie. De plus, cette technique présente l'inconvénient d'éliminer de nombreuses autres petites protéines que la β2-microglobuline, puisque l'ultrafiltrat est éliminé de façon définitive.

### 3. Couplage colonne/hémodialyseur

Cette méthode a été présentée comme une alternative aux méthodes usuelles d'hémodialyse et d'ultrafiltration (Nakazawa *et al.*, Int. J. Artif. Organs, 1994, 17, 203-208). Celle-ci consiste en une adsorption en série des biomolécules sur un gel poreux de cellulose (350 ml d'adsorbant), suivie d'une hémodialyse classique. Dans le cas de la β2-microglobuline le gel est décrit pour avoir une capacité théorique pour la β2-microglobuline de 1 mg par ml d'adsorbant. Les résultats obtenus sont les meilleurs décrits dans la littérature, puisque chez un patient dont le taux initial en β2-microglobuline était de 30 mg/l, ce système a permis de réduire la concentration en β2-microglobuline à 10 mg/l finale après 6 mois de traitement. Les auteurs ont présenté une amélioration du retardement de l'apparition des dépôts amyloïdes dans 2 cas sur 3, chez leurs patients après thérapie.

Cependant on observe également après traitement une chute de concentration de certaines molécules sériques (« rétinol binding protein », lysozymes). Ce phénomène est attribuable au passage directe du sang à travers l'adsorbant, qui est susceptible de générer des problèmes de biocompatibilité.

Ainsi les techniques existantes pour l'élimination de la β2-microglobuline et des autres biomolécules ont principalement deux limites :
- la biocompatibilité des supports, notamment pour la génération de la β2-microglobuline, c'est-à-dire l'équilibre entre l'adsorption non spécifique sur la membrane et la génération de la β2-microglobuline lors du passage des cellules à leur contact ; cet équilibre conditionne la quantité de β2-microglobuline réellement éliminée lors d'une séance d'hémodialyse ou d'hémofiltration,
- la spécificité du substrat : en effet, les techniques d'hémofiltration et de liaison aspécifique avec des ligands couplés sur des gels conduisent à l'élimination non souhaitée d'autres molécules du sérum.

Un dispositif pour éliminer la β2-microglobuline ou toute autre biomolécule devrait donc allier une élimination satisfaisante (quantitative) à une élimination spécifique (qualitative) de la molécule concernée.

Dans la présente invention, les inventeurs se sont donc donné pour objet :
- l'utilisation, dans un dispositif destiné à éliminer les biomolécules, d'un gel adsorbant alliant les propriétés des chromatographies d'exclusion stérique et d'affinité, ledit gel consistant essentiellement en une matrice de polysaccharide sur laquelle est greffé un polymère couplé à un ligand d'affinité (gel AdSEC, pour "*Adsorptive Size Exclusion Chromatography*") et ayant un seuil de coupure ajustable compris entre 2 kDa et 60 kDa,
- l'utilisation d'un gel AdSEC pour séparer et purifier les biomolécules ayant un poids moléculaire compris entre 2 kDa et 60 kDa,
- un dispositif destiné à l'élimination de biomolécules d'un poids moléculaire compris entre 2 kDa et 60 kDa comprenant un module d'ultrafiltration éventuellement en amont et en série avec un module de dialyse et utilisant une colonne de gel AdSEC ayant un seuil de coupure ajustable compris entre 2 kDa et 60 kDa, ladite colonne étant montée en dérivation dudit module d'ultrafiltration ; ce dispositif permet de s'affranchir des problèmes de biocompatibilité et d'éliminer spécifiquement les biomolécules désirées,
- un dispositif de purification de biomolécules d'un poids compris entre 2 kDa et 60 kDa utilisant une colonne de gel AdSEC ayant un seuil de coupure ajustable compris entre 2 kDa et 60 kDa, ladite colonne étant éventuellement en dérivation d'un système de filtration ; ce dispositif permet de séparer les biomolécules normales et les biomolécules modifiées, par exemple par glycation.

Dans un mode avantageux de réalisation, la matrice de polysaccharide est de l'agarose ou à base d'un dérivé d'agarose, le polymère peut être le polyéthylèneglycol (PEG) ou le polypropylèneglycol (PPG) et le ligand d'affinité peut être par exemple un agent chélateur de métaux couplé à des ions métalliques, une protéine, un peptide, un substrat d'enzyme ou un inhibiteur d'enzyme.

Dans un mode préféré de réalisation, le gel adsorbant consiste en une matrice à base d'un dérivé d'agarose sur laquelle est greffé du polyéthylèneglycol couplé à l'acide iminodiacétique (IDA) lui-même couplé à des ions métalliques, par exemple des ions cuivreux ; ce complexe est appelé gel IMAdSEC ("*Immobilized Metal ion Adsorptive Size Exclusion Chromatography*").

Dans un mode également préféré de réalisation, le seuil de coupure du gel adsorbant est de 20 kDa, permettant ainsi l'élimination ou la purifiation des biomolécules dont le poids moléculaire est inférieur à 20 kDa, en particulier la β2-microglobuline sérique.

Le système d'épuration selon la présente invention possède la particularité de placer le gel adsorbant pour la biomolécule à éliminer en dérivation du système de circulation à épurer. Ainsi lorsqu'on épure du sang, il n'y a à aucun moment contact entre le gel et les éléments figurés du sang, donc les problèmes de biocompatibilité (par exemple, génération de β2-microglobuline par contact des cellules nucléées du sang) ou d'hémolyse des cellules au contact du gel sont évités.

De plus, contrairement aux autres techniques utilisées actuellement, l'épuration de la biomolécule à éliminer ou à purifier est réalisée grâce à un ligand qui ne va retenir que cette molécule. Cette spécificité est obtenue grâce au double tamisage de la membrane d'ultrafiltration (qui retient par exemple les éléments figurés du sang et les grosses molécules sériques) et du gel AdSEC qui interdit l'accès au ligand à d'autres molécules affines pour le ligand d'affinité mais dont la taille est supérieure au seuil de coupure du gel.

L'autre intérêt de l'utilisation de ce gel AdSEC est sa facilité de régénération. Par exemple, lorsqu'on utilise un métal comme ligand d'affinité, celui-ci peut être chélaté par une solution d'EDTA, qui permet de décrocher toute molécule adsorbée sur le gel, autorisant ainsi un nettoyage du gel, sa régénération par une nouvelle charge du métal et sa stérilisation.

Le système d'élimination selon l'invention peut être utilisé par exemple dans le cadre d'une dialyse rénale ; dans ce cas il existe un avantage supplémentaire qui tient au fait que la fraction épurée par passage sur le gel AdSEC retourne au malade, limitant ainsi les pertes en autres éléments présents dans le sang.

Outre les dispositions qui précèdent, l'invention comprend encore d'autres dispositions, qui ressortiront de la description qui va suivre, qui se réfère à des exemples ainsi qu'aux figures annexées dans lesquelles :
- la figure 1 représente le schéma général de la dialyse rénale ; (1) hémodialyseur, (2) générateur d'hémodialyse, (3) pompe,
- la figure 2 représente le schéma d'une hémofiltration par ultrafiltration ; (1) hémofiltre, (2) générateur d'hémodialyse, (3) liquide physiologique, (4) pompe,

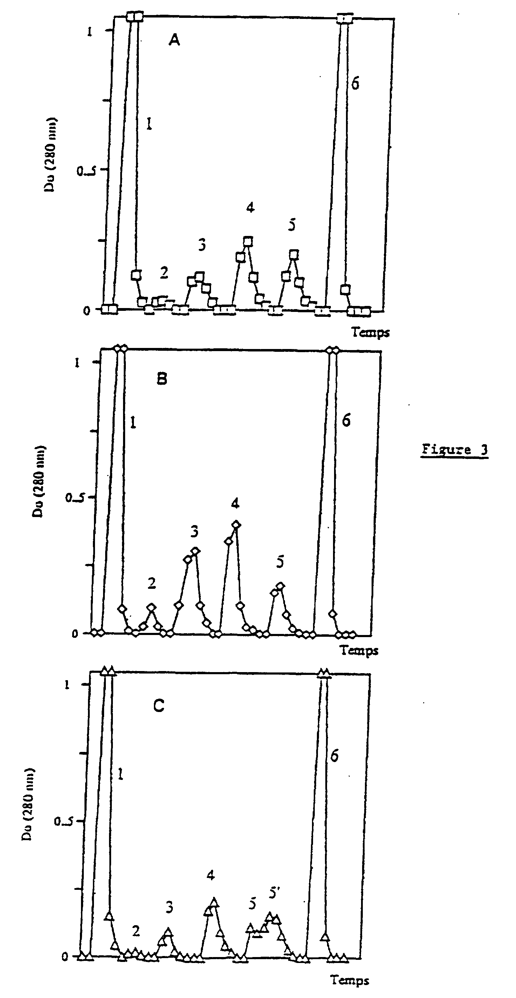

- la figure 3 illustre les chromatographies sur ions métalliques (cuivre) immobilisés sur 3 types de gels : **A** Sépharose® 4B-IDA-cuivre, pic 1 : protéines non adsorbées ; pic 2 : élution à pH 6,0 ; pic 3 : élution à pH 5,0 ; pic 4 : élution à pH 4,0 ; pic 5 : élution à pH 3,0 ; pic 6 : EDTA 25 mM. **B** Novarose®-IDA-cuivre, pic 1 : protéines non adsorbées ; pic 2 : élution à pH 6,0 ; pic 3 : élution à pH 5,0 ; pic 4 : élution à pH 4,0 ; pic 5 : élution à pH 3,0 ; pic 6 : EDTA 25 mM. C Novarose®-PEG/IDA-cuivre (IMAdSEC), pic 1 : protéines non adsorbées ; pic 2 : élution à pH 6,0 ; pic 3 : élution à pH 5,0 ; pic 4 : élution à pH 4,0 ; pic 5 : élution à pH 3,0 (1^{ier} pic) ; pic 5' élution à pH 3,0 (2^{ième} pic) ; pic 6 : EDTA 25 mM,
- la figure 4 illustre l'analyse électrophorétique des fractions séparées par chromatographie illustrée figure 3 ; les numéros correspondent aux fractions séparées par chromatographie de la figure 3 ; **A** Sépharose® 4B-IDA-cuivre. **B** Novarose®-IDA-cuivre. **C** Novarose®-PEG/IDA-cuivre (IMAdSEC). Cette figure illustre la spécificité du gel IMAdSEC pour la β2-microglobuline par rapport aux deux autres types de gels,

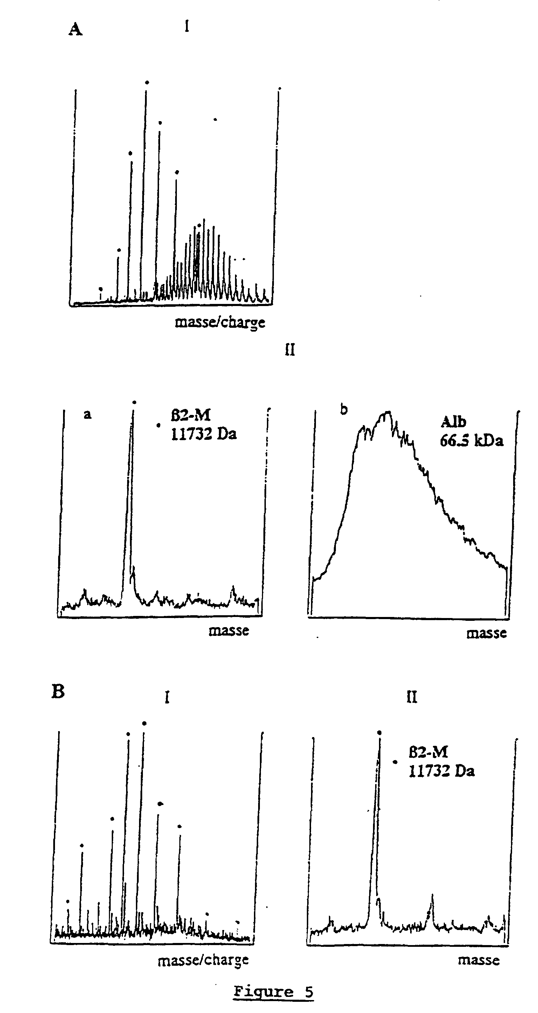

- la figure 5 illustre l'analyse par spectrométrie de masse de la composition protéique de l'ultrafiltrat de départ et de la fraction retenue sur gel IMAdSEC. **A** (I) spectre de l'ultrafiltrat, (II) déconvolution du spectre (a) calcul de la masse de la β2-microglobuline (b) calcul de la masse de l'albumine. **B** (I) spectre de la fraction purifiée, (II) déconvolution du spectre et calcul de la masse de la β2-microglobuline,
- la figure 6 illustre la capacité du gel IMAdSEC pour la β2-microglobuline,
- la figure 7 illustre le montage en dérivation d'un module de filtration du dispositif de purification selon l'invention ; (1) module d'ultrafiltration, (2) colonne contenant le gel IMAdSEC, (3) pompes, (4) ultrafiltrat,
- la figure 8 illustre la capacité du dispositif illustré figure 7 pour l'élimination de la β2-microglobuline d'un l'ultrafiltrat d'un patient urémique,
- la figure 9 illustre l'analyse électrophorétique des fractions séparées par chromatographie illustrée figure 8 ; **1** : ultrafiltrat ; **2** : 15 minutes de passage sur le gel IMAdSEC ; **3** : 30 minutes de passage sur le gel IMAdSEC ; **4** : 120 minutes de passage sur le gel IMAdSEC ; **5** : fraction éluée à pH 5,0 ; **6** : fraction éluée à pH 4,0 ; **7** fraction éluée à pH 3,0 ; **8** : faction éluée avec EDTA, **9** : standard protéique,

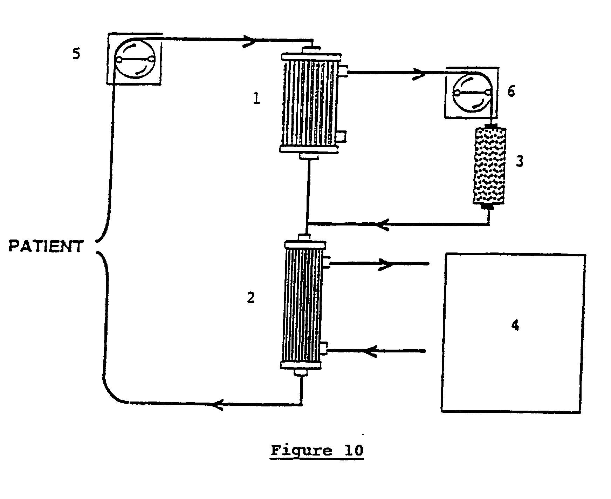

- la figure 10 représente un système d'hémodialyse comprenant le dispositif selon l'invention ; (1) hémofiltre, (2) hémodialyseur, (3) colonne IMAdSEC, (4) générateur d'hémodialyse, (5) pompe sanguine et (6) pompe d'ultrafiltration.

### EXEMPLE 1

### Détermination de la spécificité et de la capacité d'un gel IMAdSEC : (Novarose®-PEG/IDA-cuivre) pour la β2-microglobuline

### 1. Synthèse du gel Novarose®-PEG/IDA-cuivre :

Etape 1 : couplage du PEG et création du seuil de coupure du gel :

On reprend 10 g de Novarose® Act High 100/40 (INOVATA, Bromma, Suède), préalablement séchés par succion, dans 5 ml de Na₂CO₃ 1M, pH>12 et 5 ml d'eau désionisée. On ajoute 5 ml de Na₂CO₃ 1M, pH>12, 5 ml d'eau désionisée et 30 ml de NH₂-PEG-NH₂ à 10% dans du Na₂CO₃ 1M, pH>12. On laisse le mélange sous agitation douce à température ambiante (22°C) pendant 1 à 24 heures suivant le seuil de coupure souhaité (ce temps est de 4 heures pour un seuil de coupure de 20 kDa qui est le seuil souhaité pour la β2-microglobuline).

Etape 2 : couplage du ligand : l'acide iminodiacétique (IDA).

On rince le gel obtenu à l'étape 1 sur fritté (par succion) par une solution d'eau désionisée. On le resuspend dans une solution comprenant 15 ml de Na₂CO₃ 1M, pH>12, 15 ml d'eau désionisée, et 10 ml d'une solution d'IDA à 10% dans Na₂CO₃ 1M, pH>12. On laisse le mélange sous agitation douce à température ambiante (22°C) pendant 48 heures. On rince le gel IMAdSEC sur fritté successivement par de l'eau désionisée, par une solution de soude 1M, par de l'eau désionisée, par une solution d'acide chlorhydrique 0,1M, puis par de l'eau désionisée. On conserve le gel ainsi obtenu à 4°C dans une solution d'éthanol à 20% jusqu'à son utilisation.

Etape 3 : couplage des ions métalliques (ions cuivres Cu II) :

La charge de métal est réalisée en utilisant une solution aqueuse de sulfate de cuivre à 50 mM dans des conditions classiques.

### 2. Préparation des solutions biologiques

Les produits sont issus de l'hémofiltration du sang lors de séance d'ultrafiltration dans le cadre du traitement de patients urémiques (figure 2). On utilise des ultrafiltrats (pH 7,2, 13 mS/cm) dont la concentration en β2-microglobuline varie de 7 à 20 mg/l selon les patients.

### 3. Spécificité du gel Novarose®-PEG/IDA-cuivre pour la β2-microglobuline par rapport à des gels sans tamisage Sépharose®4B-IDA-cnivre et Novarose®-IDA-cuivre.

### MODE OPERATOIRE :

On a testé 3 gels : Sépharose® 4B-IDA-cuivre, Novarose®-IDA-cuivre (gels IMAC) et Novarose®-PEG /IDA-cuivre (gel IMAdSEC), pour leur capacité à adsorber les molécules de l'ultrafiltrat d'un patient urémique. Le gel de Sépharose® 4B-IDA a été préparé selon le protocole décrit par Sundberg et Porath (J. Chromatogr., 1974, 90, 87-98). Le gel Novarose®-IDA résulte du même protocole que celui décrit ci-dessus au point 1 pour la synthèse du gel IMAdSEC, où seule la deuxième et la troisième étape ont été réalisées (pas d'activation préalable du gel par le PEG). On applique 2 ml de gel dans une colonne (diamètre 1 cm) et on réalise une chromatographie basse pression (1 ml/min). On fait passer 10 ml d'ultrafiltrat d'un patient, dont la concentration en β2-microglobuline est de 20 µg/ml sur chacun des 3 différents gels en circuit fermé durant 20 minutes. On réalise l'équilibration et le rinçage de chaque colonne après adsorption de l'ultrafiltrat par un tampon MMA pH 7,0 (MMA = MOPS, MES, Acétate, 25 mM chacun). On élue par un gradient décroissant discontinu de pH (Tampon, MMA 25 mM, pH 6,0, puis pH 5,0, puis pH 4,0 et glycine 25 mM à pH 3,0), puis par une solution d'EDTA (50 mM) pour décrocher le cuivre. On mesure la teneur en protéines lors de la chromatographie par lecture de la densité optique (λ = 280 nm) avec un détecteur placé en sortie de colonne. On réalise le dosage de la β2-microglobuline par un dosage immunologique (Anticorps polyclonal de lapin anti β2-microglobuline humaine, Dako, Danemark) utilisant un appareil de néphélémétrie (Beckman, USA). On analyse les différentes fractions par électrophorèse SDS-PAGE, selon le protocole décrit par Laemmli (Nature, 1970, 227, 680-685) et coloration des protéines au nitrate d'argent. Après dessalage et concentration, on analyse les fractions par spectrométrie de masse (technique ESI-MS pour "*ElectroSpray Ionisation Mass Spectrometry"),* dont la sensibilité, déterminant la masse au Dalton près, permet d'identifier les molécules.

### RESULTATS :

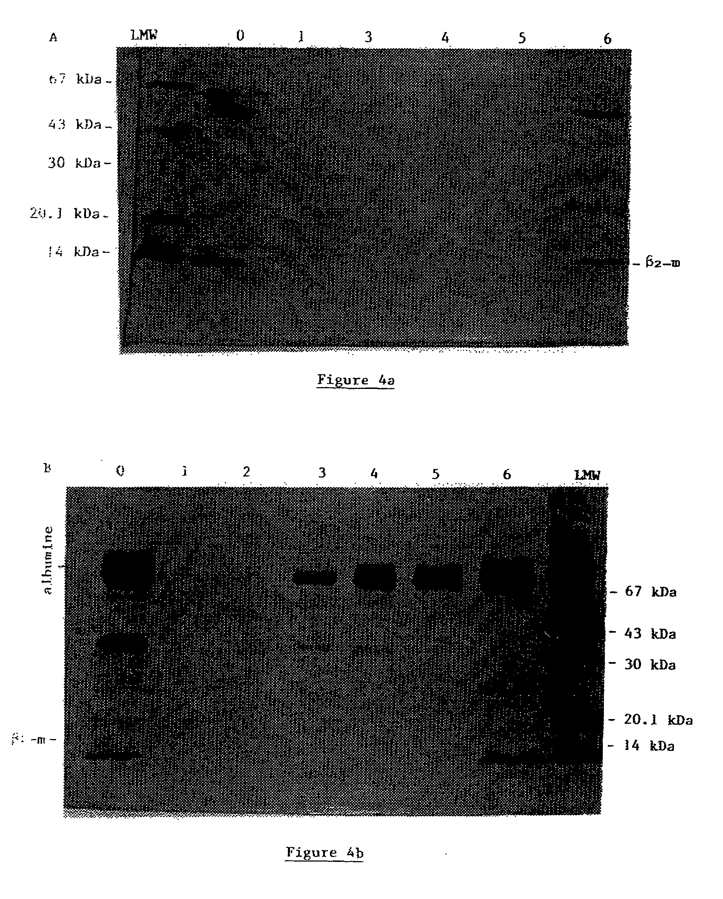

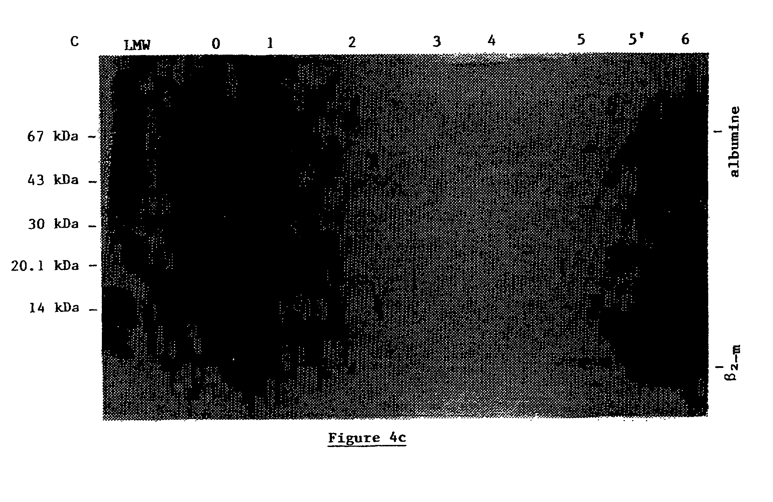

* La chromatographie sur gel de Sépharose® 4B-IDA-cuivre (figure 3A) montre que, si la β2-microglobuline présente une affinité importante pour le cuivre chélaté, son élution a lieu dans les mêmes fractions que l'albumine (figure 4A). Toutes les protéines de l'ultrafiltrat sont adsorbées sur le gel, qui ne présente donc pas de spécificité pour la β2-microglobuline.
* La chromatographie sur gel Novarose®-IDA-cuivre (figure 3B) montre également que ce type de gel permet l'adsorption de toutes les protéines de l'ultrafiltrat (figure 4B). Sa capacité en cuivre plus faible que celle du Sépharose®-4B-IDA résulte en revanche en des élutions de protéines lors du gradient discontinu de pH, contrairement au gel Sépharose® 4B-IDA (figure 4B versus 4A). Comme ce dernier, il n'assure pas de spécificité pour la β2-microglobuline (figure 4B).
* La chromatographie sur gel Novarose®-PEG/IDA-cuivre en revanche a permis l'adsorption de la seule β2-microglobuline de l'ultrafiltrat du patient. Son élution a lieu à pH 3,0 en deux pics distincts (figure 4C).

Dans les trois types de chromatographie, les analyses par néphélémétrie confirment la disparition totale de la β2-microglobuline de la fraction d'ultrafiltrat passée sur les 3 types de gels et son élution de la colonne.

L'analyse ESI-MS montre que la chromatographie sur gel IMAdSEC permet de passer d'une fraction constituée d'un mélange de départ : albumine + β2-microglobuline, à une fraction éluée à pH 3,0 qui contient uniquement la β2-microglobuline (figure SA versus 5B).

Ces résultats montrent l'affinité de la β2-microglobuline pour le ligand (métal chélaté, ici le cuivre) et la spécificité apportée par le tamisage moléculaire (couplage du PEG) du gel IMAdSEC par rapport aux gels IMAC classiques.

### 4. Capacité du gel IMAdSEC-cuivre pour la β2-microglobuline.

### MODE OPERATOIRE :

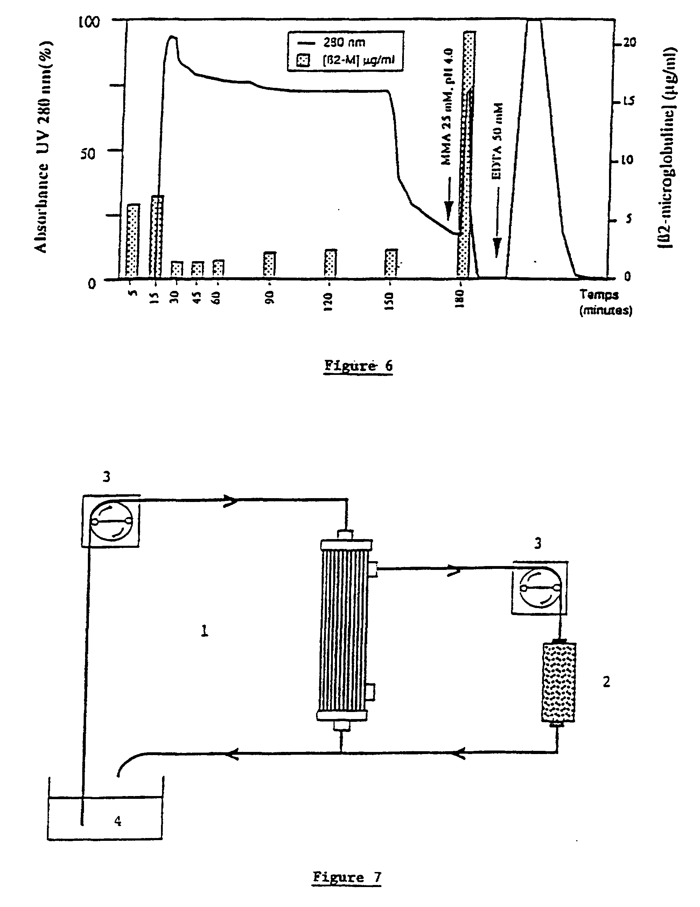

50 ml d'ultrafiltrat d'un patient urémique, contenant 350 µg de β2-microglobuline (soit une concentration de β2-microglobuline de 7 µg/ml) circule en circuit fermé pendant 150 minutes sur 0,65 ml de gel IMAdSEC dans les mêmes conditions chromatographiques que précédemment (débit = 1 ml/min). On élue directement à pH 4,0 (figure 6).

### RESULTATS :

Après 150 minutes, la concentration en β2-microglobuline mesurée par néphélémétrie est de 2,3 µg/ml, soit une quantité en β2-microglobuline restante de 115 µg. Par conséquent, 235 µg de β2-microglobuline ont été fixés sur les 0,65 ml de gel, ce qui correspond à une capacité de fixation du gel IMAdSEC-cuivre de 360 µg/ml. L'analyse SDS-PAGE et ESI-MS des fractions a été réalisée comme décrit précédemment. La quantité de β2-microglobuline, éluée à pH 4,0, est d'environ 180 µg au lieu de 235 µg attendus. La différence peut être expliquée par l'absence de mesure des fractions de rinçage et d'EDTA susceptibles de contenir elles aussi de la β2-microglobuline.

Ces résultats suggèrent que, compte-tenu de ces performances et de cette spécificité pour la β2-microglobuline, une colonne de 500 à 750 ml de gel IMAdSEC-cuivre permettrait d'éliminer 250 mg de β2-microglobuline, quantité qui correspond à 5 litres de sang à une concentration en β2-microglobuline de 50 mg/l.

### EXEMPLE 2

### Séparation et purification de la β2-microglobuline par un dispositif comprenant le couplage d'un module d'ultrafiltration et d'une colonne IMAdSEC

### MODE OPERATOIRE

On utilise le montage représenté figure 7. Le module d'ultrafiltration (1) utilisé est composé de 100 fibres creuses en Polysulfone tirées d'un module d'ultrafiltration commercial modèle Fresenius F80.

On passe 50 ml d'ultrafiltrat d'un patient urémique (concentration en β2-microglobuline = 7 µg/ml) en circuit fermé durant 3 heures sur l'ensemble mini-module d'ultrafiltration/colonne de gel IMAdSEC (0,65 ml de gel IMAdSEC). Les conditions de cromatographie sont celles de l'exemple 1 à savoir : tampon, MMA 25 mM, pH 6,0, puis pH 5,0, puis pH 4,0 et glycine 25 mM à pH 3,0, puis EDTA 50 mM pour éluer le cuivre chélaté sur le gel.

Après 3 heures, on mesure la concentration en β2-microglobuline dans le réservoir par néphélémétrie.

### RESULTATS

On passe d'une concentration de 7 µg/ml en β2-microglobuline (soit une quantité de départ de 350 µg) à environ 1 µg/ml (50 µg de β2-microglobuline restante). Par conséquent environ 300 µg de β2-microglobuline ont été fixés sur les 0,65 ml de gel IMAdSEC, ce qui correspond à une capacité de fixation du gel IMAdSEC pour la β2-microglobuline de 461 µg/ml.

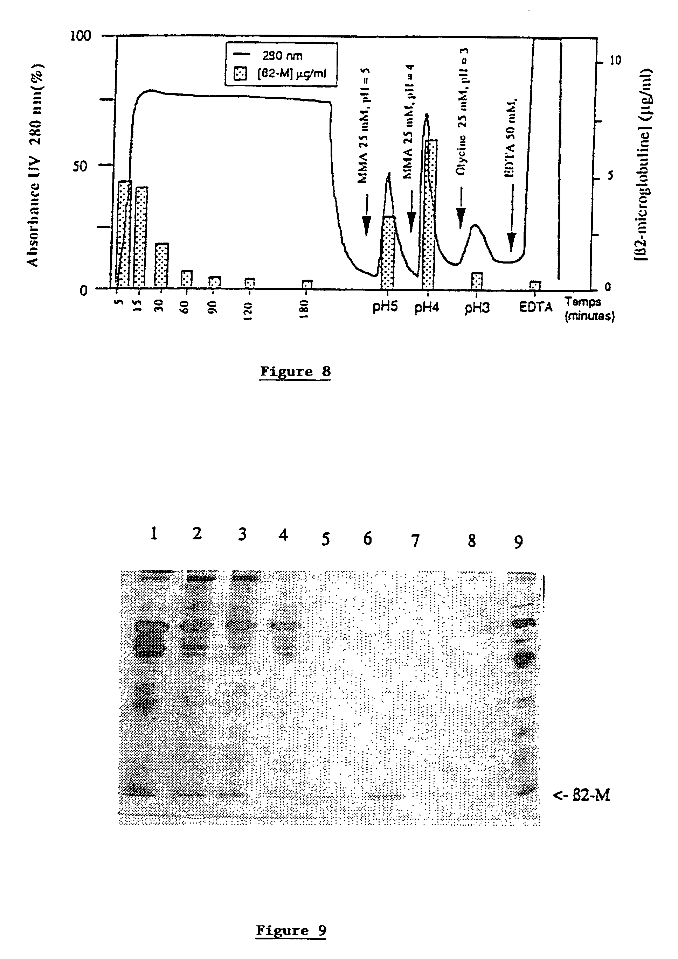

L'analyse ESI-MS (figure 8) et SDS-PAGE (figure 9) des fractions montrent que la β2-microglobuline a été adsorbée de façon spécifique par le gel IMAdSEC. Elle est éluée en deux fractions principales à pH 4,0 et pH 5,0.

Ces résultats suggèrent que le gel IMAdSEC pourrait être utile pour la séparation des biomolécules et de leurs isoformes comme par exemple la β2-microglobuline normale et la β2-microglobuline glycatée.

## Revendications

1. Dispositif pour éliminer les biomolécules comprenant un module d'ultrafiltration éventuellement en amont et en série d'un module de dialyse, **caractérisé en ce que** ce dispositif comprend en plus une colonne contenant un gel adsorbant alliant les propriétés des chromatographies d'exclusion stérique et d'affinité, ledit gel adsorbant consistant essentiellement en une matrice de polysaccharide sur laquelle est greffé un polymère couplé à un ligand d'affinité et ayant un seuil de coupure ajustable compris entre 2 kDa et 60 kDa, ladite colonne étant montée en dérivation dudit module d'ultrafiltration.

2. Dispositif selon la revendication 1, **caractérisé en ce que** le gel adsorbant consiste en une matrice à base d'un dérivé d'agarose sur laquelle est greffé du polyéthylèneglycol couplé à l'acide iminodiacétique lui-même couplé à des ions cuivreux et ayant un seuil de coupure de 20 kDa.

3. Dispositif pour séparer et purifier des biomolécules comprenant une colonne contenant un gel adsorbant alliant les propriétés des chromatographies d'exclusion stérique et d'affinité, ledit gel consistant essentiellement en une matrice de polysaccharide sur laquelle est greffé un polymère couplé à un ligand d'affinité et ayant un seuil de coupure ajustable compris entre 2 kDa et 60 kDa, ladite colonne étant éventuellement montée en dérivation d'un module d'ultrafiltration.

4. Dispositif selon la revendication 3, **caractérisé en ce que** le gel adsorbant consiste en une matrice à base d'un dérivé d'agarose sur laquelle est greffé du polyéthylèneglycol couplé à l'acide iminodiacétique lui-même couplé à des ions cuivreux et ayant un seuil de coupure de 20 kDa.

5. Dispositif selon la revendication 2 ou la revendication 4, **caractérisé en ce que** la biomolécule est la β2-microglobuline.

6. Dispositif selon l'une quelconque des revendications 1 à 5, **caractérisé en ce que** le dispositif est un système de dialyse extra-corporelle.

## Patentansprüche

1. Vorrichtung zum Entfernen von Biomolekülen, mit einem gegebenenfalls stromaufwärts von und in Reihe mit einem Dialysemodul angeordneten Ultrafiltrationsmodul, **dadurch gekennzeichnet, daß** die Vorrichtung des weiteren eine Säule aufweist, die ein adsorbierendes Gel enthält, welches die Eigenschaften der sterischen Flüssig-Ausschlußchromatographie und der Affinitätschromatographie vereint, wobei das adsorbierende Gel im wesentlichen aus einer Polysaccharidmatrix besteht, auf die ein Polymer aufgepropft ist, das an einen Affinitätsliganden gekoppelt ist, und einen zwischen 2 kDa und 60 kDa liegenden, einstellbaren Cutoff-Schwellwert aufweist, wobei die Säule vom Ultrafiltrationsmodul abzweigend angebracht ist.

2. Vorrichtung nach Anspruch 1, **dadurch gekennzeichnet, daß** das adsorbierende Gel aus einer Matrix auf Basis eines Agarosederivats besteht, auf die Polyethylenglycol aufgepropft ist, welches an Iminodiessigsäure gekoppelt ist, welche wiederum an Kupferionen gekoppelt ist, und einen Cutoff-Schwellwert von 20 kDa aufweist.

3. Vorrichtung zum Abtrennen und Reinigen von Biomolekülen, mit einer Säule, die ein adsorbierendes Gel enthält, welches die Eigenschaften der sterischen Flüssig-Ausschlußchromatographie und der Affinitätschromatographie vereint, wobei das Gel im wesentlichen aus einer Polysaccharidmatrix besteht, auf die ein Polymer aufgepropft ist, das an einen Affinitätsliganden gekoppelt ist, und einen zwischen 2 kDa und 60 kDa liegenden, einstellbaren Cutoff-Schwellwert aufweist, wobei die Säule gegebenenfalls von einem Filtrationsmodul abzweigend angebracht ist.

4. Vorrichtung nach Anspruch 3, **dadurch gekennzeichnet, daß** das adsorbierende Gel aus einer Matrix auf Basis eines Agarosederivats besteht, auf die Polyethylenglycol aufgepropft ist, welches an Iminodiessigsäure gekoppelt ist, welche wiederum an Kupferionen gekoppelt ist, und einen Cutoff-Schwellwert von 20 kDa aufweist.

5. Vorrichtung nach Anspruch 2 oder 4, **dadurch gekennzeichnet, daß** das Biomolekül serisches β2-Mikroglobulin ist.

6. Verwendung der Vorrichtung nach den Ansprüchen 1 bis 5 zum Entfernen von Biomolekülen aus Blut, mit Ausnahme der extrakorporalen Dialyse.

## Claims

1. Device for removing biomolecules comprising an ultrafiltration module optionally upstream and in series with a dialysis module, **characterized in that** this device further comprises a column containing an adsorbent gel combining the properties of size exclusion and affinity chromatographies, said adsorbent gel consisting essentially of a polysaccharide matrix onto which is grafted a polymer coupled to an affinity ligand and having an adjustable cut-off of between 2 kDA and 60 kDa, said column being mounted branching from said ultrafiltration module.

2. Device according to claim 1, **characterized in that** the adsorbent gel consists of a matrix based on an agarose derivative onto which is grafted polyethylene glycol coupled to iminodiacetic acid itself coupled to copper(I) ions and having a cut-off of 20 kDa.

3. Device for separating and purifying biomolecules comprising a column containing an adsorbent gel combining the properties of size exclusion and affinity chromatographies, said gel consisting essentially of a polysaccharide matrix onto which is grafted a polymer coupled to an affinity ligand and having an adjustable cut-off of between 2 kDa and 60 kDa, said column being optionally mounted branching from a fiteration mocule.

4. Device according to claim 3, **characterized in that** the adsorbent gel consists of a matrix based on an agarose derivative onto which is grafted polyethylene glycol coupled to iminodiacetic acid itself coupled to copper(I) ions and having a cut-off of 20 kDa.

5. Device according to claim 2 or claim 4,
**characterized in that** the biomolecule is serum β2-microglobulin.

6. Use of the device according to claims 1 to 5 for removing biomolecules from blood, with the exception of extracorporeal dialysis.
